# DEMANDE DE BREVET EUROPEEN

(11) **EP 0 945 105 A1**
(43) Date de publication de la demande: **29.09.1999**
(21) Numéro de dépôt: 99105450.3
(22) Date de dépôt: 17.03.1999
(51) Int. Cl.: A61B 17/28

(54) **Dispositif de prélèvement, par exemple pour biopsie, et système à crémaillère équipant un tel dispositif**

(30) Priorité: 26.03.1998 EP 98105473
(71) Demandeur: Nivarox-FAR S.A., CH-2400 Le Locle (CH)
(72) Inventeur: Peters, Jean-Bernard, 2300 La Chaux-de-Fonds (CH)
(74) Mandataire: Ravenel, Thierry Gérard Louis

(57) **Abrégé**

La présente invention concerne un dispositif de prélèvement, par exemple pour biopsie, du type comprenant un système de commande mécanique (2) coulissant à l'intérieur d'une gaine souple (4) et reliant une poignée de commande à une pince (6) formée par des mâchoires pivotantes (8), caractérisé en ce que le système de commande (2) comprend une crémaillère cylindrique (28), et en ce que les mâchoires (8) de la pince (6) présentent chacune sur une partie circulaire de leur périmètre une denture (34) engageant la crémaillère cylindrique (28) pour l'ouverture et la fermeture desdites mâchoires (8).

## Description

La présente invention concerne un dispositif de prélèvement, par exemple pour biopsie, du type comprenant un câble de commande coulissant à l'intérieur d'une gaine souple, et une pince formée par au moins deux mâchoires ou mors pivotants. L'invention concerne également un système à crémaillère à transmission de couple mécanique améliorée entre un pignon menant et un pignon mené.

Les pinces à biopsie sont notamment utilisées pour effectuer chez un patient des prélèvements de tissus sur des organes vivants, par exemple en vue d'examens cliniques. Ces pinces comprennent classiquement des mâchoires coupantes actionnées à distance par un manipulateur au moyen d'un dispositif de commande comprenant une poignée reliée aux mâchoires par un système de câbles coulissants dans une gaine souple. La pince et sa gaine souple sont introduites dans le tube d'un endoscope par l'intermédiaire duquel la pince est amenée à l'emplacement de prélèvement souhaité.

Ce matériel est utilisé par un personnel qualifié qui, bien qu'averti de la fragilité de ces instruments, ne peut éviter la rupture très fréquente des câbles qui assurent l'ouverture et la fermeture des mâchoires desdits instruments. Il faut savoir, en effet, que ces pinces, en raison de leur destination et de leur mode d'emploi, sont de dimensions très réduites et que leurs câbles sont extrêmement fins. D'autre part, il faut remarquer que la pression que l'on doit exercer sur la poignée pour assurer le mouvement de fermeture des mâchoires doit être assez forte pour pouvoir prélever en découpant des fragments de tissus ou de muqueuses. Cette pression ne peut toutefois se mesurer et se contrôler avec précision, de sorte qu'elle est laissée à l'appréciation du manipulateur qui ne l'évalue pas toujours correctement, ce qui est la cause de la rupture des câbles.

Diverses solutions ont été proposées pour tenter de remédier à ces problèmes et assurer un prélèvement plus précis et plus efficace.

A titre d'exemple, on peut citer le brevet US 5,582,617 qui propose une pince à biopsie du type comprenant deux mâchoires articulées à pivotement autour d'un axe commun, ces mâchoires présentant chacune un profil denté circulaire engageant une crémaillère rectiligne fixée par sertissage sur le câble de commande de la pince. Par traction sur le câble, les mâchoires de la pince se referment.

Une autre forme de réalisation d'une pince est connue du brevet US 5,209,747. Cette pince comprend une tige de commande présentant deux crémaillères rectilignes qui engagent chacune le profil circulaire denté de l'une des mâchoires de la pince.

Ces solutions connues présentent plusieurs inconvénients. On remarquera tout d'abord que le couple mécanique est transmis aux mâchoires ou pignons menés des pinces par une seule des dents des crémaillères rectilignes ou pignons menants. La pression exercée par le manipulateur sur la poignée n'est donc que partiellement transmise aux mâchoires des pinces, de sorte qu'il existe un risque important de déchirer et d'arracher les tissus à prélever. On peut également considérer le fait que de telles pinces ne peuvent comporter plus de deux mâchoires et que leur volume de prélèvement est donc limité. Enfin, de telles pinces sont généralement peu sûres. En cas de rupture du câble de commande ou de lâchage du sertissage, les crémaillères risquent d'échapper et de tomber dans l'organisme du patient.

On connaît également par le brevet US 5,275,615 une pince à biopsie comprenant un câble de transmission coulissant à l'intérieur d'une gaine souple et reliant une poignée de commande à une pince formée par deux mâchoires pivotantes. Selon l'invention décrite dans ce document, la pince comprend une tige de commande cylindrique présentant deux crémaillères semi-circulaires qui engagent chacune le profil circulaire denté de l'une des mâchoires de la pince. Une telle pince ne peut donc comporter plus de deux mâchoires, ce qui limite son volume de prélèvement. On remarquera d'autre part que l'engrenage utilisé dans ce brevet est un engrenage normalisé avec au moins douze dents apparentes réparties sur le diamètre primitif du profil circulaire denté de chacune des mâchoires de la pince. Or, les inconvénients des dentures normalisées sont bien connus dans le domaine de la micromécanique : mauvaise conduite d'engrenage, fragilité des dents, mauvaise transmission du couple mécanique exercé par le manipulateur lorsqu'il actionne la poignée de commande.

La présente invention a pour but de remédier aux problèmes et inconvénients ci-dessus en proposant un dispositif de prélèvement, notamment pour biopsie, d'un fonctionnement sûr et permettant une bonne transmission à la pince de la pression exercée par le manipulateur sur la poignée de commande.

A cet effet, la présente invention concerne un dispositif de prélèvement, par exemple pour biopsie, du type comprenant un système de commande mécanique coulissant à l'intérieur d'une gaine souple et reliant une poignée de commande à une pince formée par des mâchoires pivotantes, caractérisé en ce que le système de commande comprend une crémaillère cylindrique, et en ce que les mâchoires de la pince présentent chacune sur une partie circulaire de leur périmètre une denture engageant la crémaillère cylindrique pour l'ouverture et la fermeture desdites mâchoires.

La crémaillère selon l'invention présentant une symétrie générale de révolution, le nombre de mâchoires de la pince peut être augmenté et porté à au moins trois. Il est ainsi possible d'augmenter le volume de prélèvement de la pince, et d'améliorer l'efficacité et la précision du prélèvement.

Selon une autre caractéristique de l'invention, les dentures des mâchoires de la pince engagent la crémaillère cylindrique selon au moins deux de leurs dents. Le couple mécanique correspondant à la pression exercée par le manipulateur sur la poignée de commande du dispositif de prélèvement selon l'invention est ainsi intégralement transmis aux mâchoires de la pince, ce qui assure un prélèvement précis et efficace, sans risque de déchirer ou d'arracher par exemple les tissus à prélever.

Selon une autre caractéristique de l'invention, les dentures des mâchoires de la pince présentent chacune une butée qui bloque le coulissement axial de la crémaillère cylindrique dans une position de fin de course. Grâce à cette autre disposition avantageuse, la crémaillère cylindrique devient imperdable, même en cas de rupture du câble de commande ou de lâchage du sertissage ou de la soudure, ce qui procure une grande sécurité d'utilisation au dispositif de prélèvement selon l'invention.

Selon encore une autre caractéristique de l'invention, le nombre apparent de dents réparties sur le diamètre primitif de l'engrenage est de six au plus. Ainsi, en divisant le nombre apparent de dents par deux par rapport à un engrenage normalisé, la présente invention permet d'obtenir un engrenage avec des dents plus solides, une meilleure conduite, ainsi qu'une meilleure transmission du couple mécanique exercé par le manipulateur lorsqu'il actionne la poignée de commande.

La présente invention concerne également un système à crémaillère comprenant un pignon menant et un pignon mené, caractérisé en ce que le pignon menant est une crémaillère cylindrique, et en ce que le pignon mené présente sur une partie circulaire de son périmètre une denture engageant la crémaillère cylindrique.

D'autres caractéristiques et avantages de la présente invention apparaîtront plus clairement à la lecture de la description détaillée qui suit d'un exemple de réalisation du dispositif de prélèvement selon l'invention, cet exemple étant donné à titre purement illustratif et non limitatif, en liaison avec les dessins annexés dans lesquels :
- la figure 1 est une vue générale en perspective de la pince de prélèvement selon l'invention en position de pleine ouverture avec arrachement partiel au niveau de la chape;
- la figure 2 est une vue en coupe longitudinale de la pince de prélèvement de la figure 1 en position de pleine ouverture;

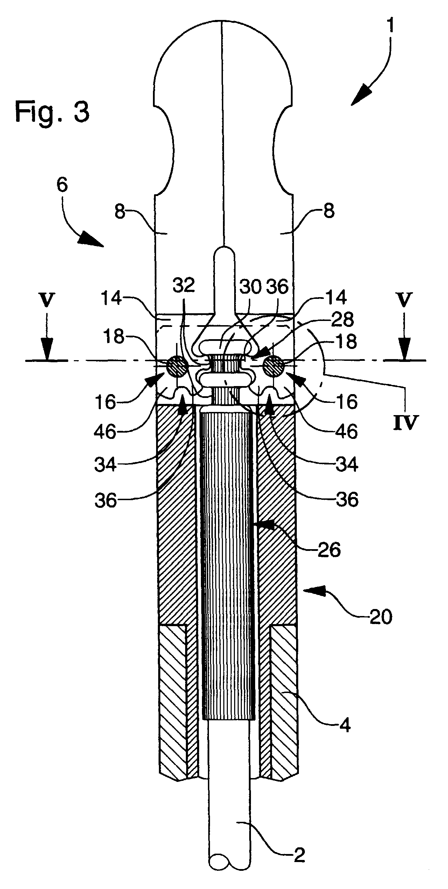

- la figure 3 est une vue en coupe longitudinale de la pince de prélèvement en position fermée;
- la figure 4 est une vue de détail de la partie entourée d'un trait mixte sur la figure 3;
- la figure 5 est une vue en coupe selon la ligne V-V de la figure 3, les mâchoires de la pince ayant été retirées, et
- la figure 6 est une vue partielle du système d'engrenage selon l'invention.

Le dispositif de prélèvement selon l'invention va être décrit dans son application médicale au prélèvement de tissus sur des organes vivants ou biopsie. Il va néanmoins de soi que ce dispositif peut être utilisé à d'autres fins pour la préhension ou le prélèvement d'échantillons, de fragments ou de particules de natures diverses, organiques, minérales ou autres.

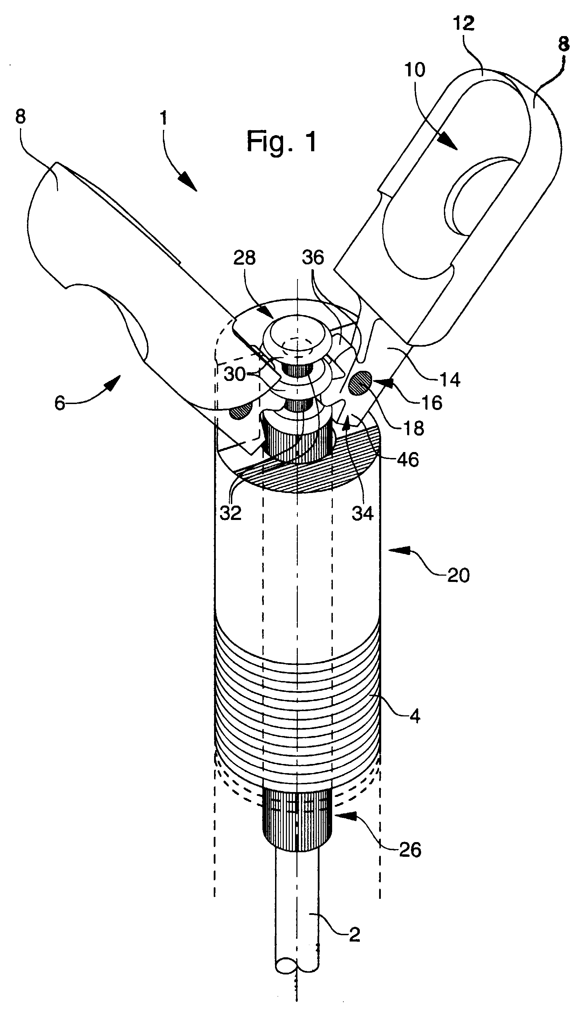

Comme il ressort notamment de la figure 1, le dispositif de prélèvement selon l'invention, désigné dans son ensemble par la référence numérique générale 1, comprend un système de commande mécanique formé par un câble de transmission 2 coulissant axialement à l'intérieur d'une gaine souple 4 et reliant une poignée de commande (non représentée) à une pince 6 formée par deux mâchoires pivotantes telles que 8. Par traction sur le câble 2, les mâchoires 8 de la pince 6 se referment.

Dans l'application de la présente invention à la réalisation de pinces à biopsie ou autres pinces destinées à effectuer des prélèvements de fragments de matière, les mâchoires 8 peuvent présenter, sur leur face interne, une cavité 10 sensiblement hémisphérique présentant vers son rebord supérieur un bord tranchant 12. Ainsi, lorsque la pince 6 est fermée, les mâchoires 8 délimitent un volume de prélèvement suffisamment important pour renfermer un fragment de tissu biologique, un fragment de minéral ou autre. Les mâchoires 8 peuvent être réalisées entièrement en acier inoxydable ou en un matériau plastique biocompatible adapté, les bords tranchants 12 étant alors rapportés sur les mâchoires 8 sous la forme d'inserts métalliques. Il va de soi qu'une importante gamme de mâchoires 8 de conformations différentes peut être proposée aux utilisateurs en fonction de leurs besoins.

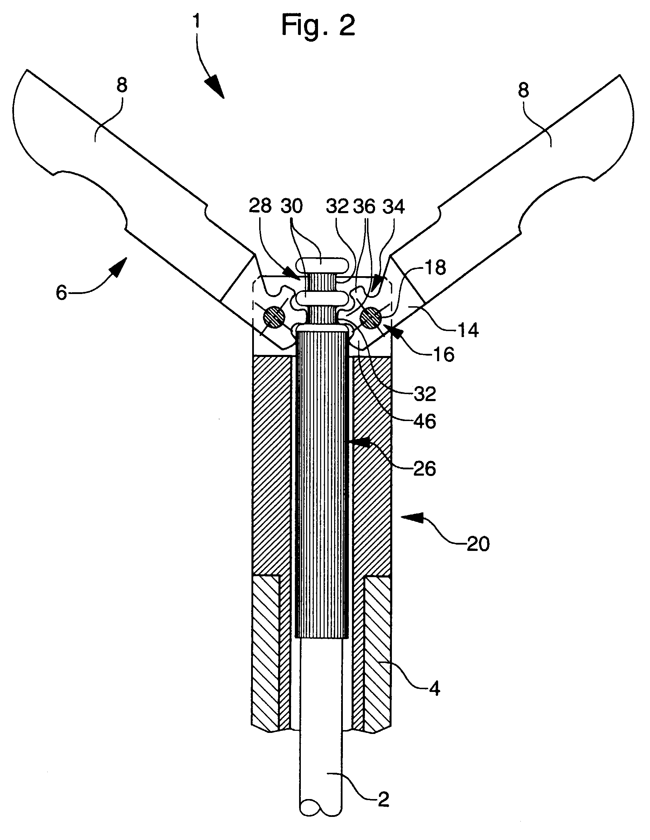

Les mâchoires 8 de la pince 6 comprennent chacune une branche de montage 14 dont l'extrémité présente un trou 16 pour le passage d'un axe 18. Comme il ressort notamment de la figure 2, les mâchoires 8 sont montées à pivotement autour des axes 18 sur une chape 20 fixée à l'extrémité terminale de la gaine souple 4 par sertissage, soudage ou toute autre technique appropriée. Cette chape 20, de forme générale extérieure par exemple cylindrique, présente une rainure diamétrale 22 dans laquelle sont logées les branches de montage 14 des mâchoires 8. Les mâchoires 8 de la pince 6 sont ainsi complètement protégées et ne risquent pas d'être endommagées lors du nettoyage, classiquement par brossage, de ladite pince 6.

Conformément à la présente invention, la chape 20 comporte également en son centre un canal longitudinal 24 pour le guidage à coulissement axial d'une tige de commande 26 fixée par sertissage, soudage ou autre à l'extrémité libre du câble de transmission 2. Selon une première caractéristique avantageuse de l'invention, la tige de commande 26 présente à son sommet une crémaillère cylindrique ou pignon menant 28 constituée par une succession alternée de dents annulaires telles que 30 et de gorges annulaires telles que 32. Les dents 30 qui se présentent sous la forme de disques pleins peuvent être avantageusement formées d'un seul tenant avec la tige de commande 26, par exemple par décolletage. La crémaillère cylindrique 28 représentée sur les figures ne comporte que deux dents 30. Il va néanmoins de soi qu'en fonction de la destination et du mode d'emploi de la pince 6 recherchés, ce nombre pourra être augmenté.

Les branches de montage 14 des mâchoires 8 présentent chacune sur une partie circulaire de leur périmètre une denture ou pignon mené 34 dont les dents telles que 36 engagent la crémaillère cylindrique 28 de sorte que, par traction sur le câble de transmission 2, les mâchoires 8 de la pince 6 se referment.

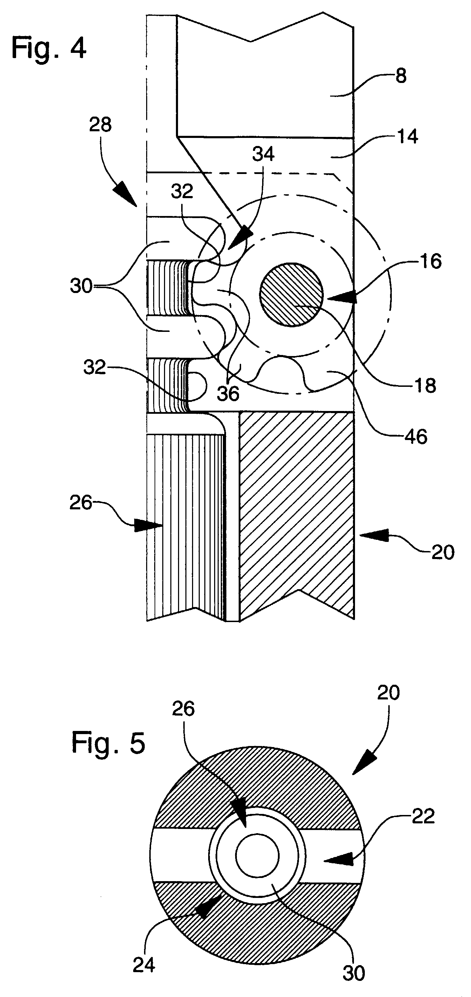

Comme il ressort clairement de la figure 4, les dentures ou pignons menés 34 engagent la crémaillère cylindrique ou pignon menant 28 selon deux de leurs dents 36. Grâce à cette caractéristique, le couple mécanique correspondant à la pression exercée par le manipulateur sur la poignée de commande du dispositif de prélèvement selon l'invention est intégralement transmis aux mâchoires 8 de la pince 6, ce qui assure un prélèvement précis et efficace, sans risque de déchirer ou d'arracher les tissus à prélever. Il faut également remarquer que la géométrie sensiblement de révolution de la crémaillère cylindrique 28 permet d'envisager d'augmenter le nombre de mâchoires 8 qui forment la pince 6, et de porter ce nombre par exemple à trois. Il est ainsi possible d'augmenter le volume de prélèvement de la pince 6 et d'accroître encore la précision et l'efficacité du prélèvement.

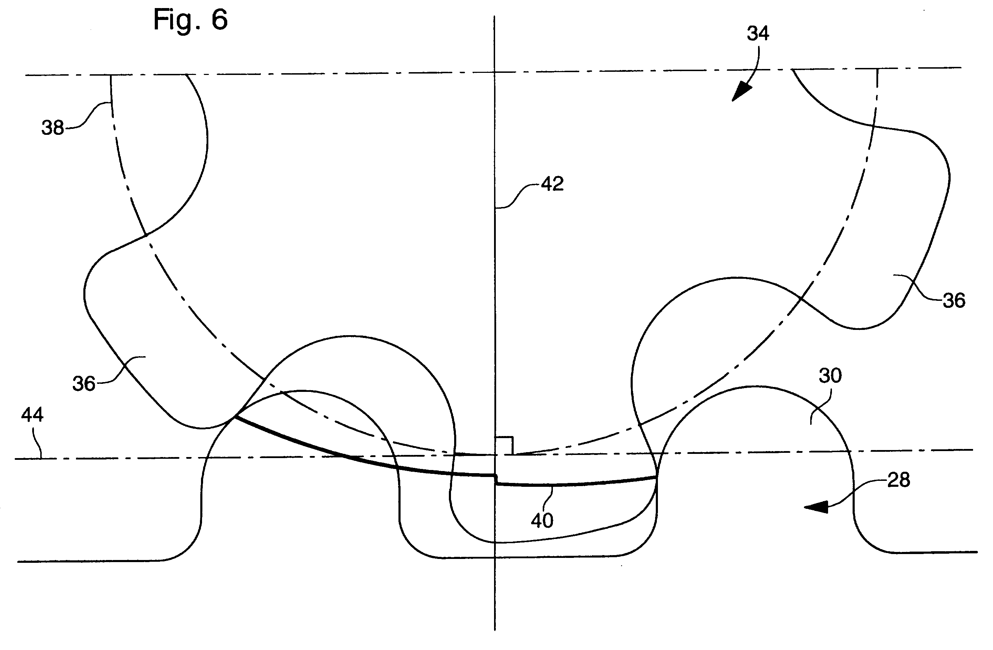

Le système à engrenage décrit ci-dessus est un système de forme spéciale présentant des dents de très forte résistance. Comme il ressort de la figure 6, le nombre apparent de dents 36 du pignon mené 34 de ce système est de six au plus, réparties sur le diamètre primitif 38 dudit pignon mené 34. Selon une caractéristique avantageuse de l'invention, la ligne 40 formée par les points de contact entre les dents 30 du pignon menant 28 et les dents 36 du pignon mené 34 est plus longue après la ligne neutre 42 (sortie de l'engrenage) qu'avant la ligne 42 (entrée de l'engrenage). Au sens de l'invention, on entend par ligne neutre 42 la droite qui passe par le centre du pignon 34 et qui est perpendiculaire à la droite 44 tangente au diamètre primitif 38 dudit pignon 34. Cette disposition permet d'éviter les points de blocage caractéristiques des engrenages normalisés à faible nombre de dents pour lesquels la ligne des points de contact est symétrique par rapport à la ligne neutre, ce qui crée un phénomène de friction à l'entrée du système à engrenage.

Selon un autre avantage de l'invention, les dentures 34 des mâchoires 8 présentent chacune une butée 46 de fin de course qui empêche le coulissement axial de la crémaillère cylindrique 28 en position de pleine ouverture de la pince 6. La crémaillère 28 devient ainsi imperdable, même en cas de rupture du câble de transmission 2, ou de lâchage de la soudure ou du sertissage par lequel la tige de commande 26 est fixée sur ledit câble de transmission 2. Les risques de voir la tige de commande 26 tomber par exemple dans l'organisme d'un patient sont ainsi supprimés, ce qui confère au présent dispositif une grande sécurité d'utilisation. Il faut également remarquer qu'en cas de rupture du câble de transmission 2, la fermeture des mâchoires 8 de la pince 6 reste malgré tout possible par simple rétraction de ladite pince 6 à l'intérieur du tube de l'endoscope, sans risque d'endommager ce dernier.

Il peut également être prévu une aiguille en extrémité de la crémaillère cylindrique 28, de manière à pouvoir planter la pince 6 en un endroit précis avant d'effectuer un prélèvement par fermeture des mâchoires 8.

Il va de soi qu'au-delà des moyens décrits, diverses modifications et variantes simples entrent dans le cadre de la présente invention.

## Revendications

1. Dispositif de prélèvement, par exemple pour biopsie, du type comprenant un système de commande mécanique (2) coulissant à l'intérieur d'une gaine souple (4) et reliant une poignée de commande à une pince (6) formée par des mâchoires pivotantes (8), caractérisé en ce que le système de commande (2) comprend une crémaillère cylindrique (28), et en ce que les mâchoires (8) de la pince (6) présentent chacune sur une partie circulaire de leur périmètre une denture (34) engageant la crémaillère cylindrique (28) pour l'ouverture et la fermeture desdites mâchoires (8).

2. Dispositif de prélèvement selon la revendication 1, caractérisé en ce que la denture (34) engage la crémaillère cylindrique (28) par au moins deux de ses dents (36).

3. Dispositif de prélèvement selon l'une quelconque des revendications précédentes, caractérisé en ce que le nombre apparent de dents (36) réparties sur le diamètre primitif (38) de la denture (34) est de six au plus.

4. Dispositif de prélèvement selon l'une quelconque des revendications précédentes, caractérisé en ce que la crémaillère cylindrique (28) est constituée par une succession alternée de dents annulaires (30) et de gorges annulaires (32).

5. Dispositif de prélèvement selon la revendication 4, caractérisé en ce que la ligne (40) formée par les points de contact entre les dents (30) de la crémaillère cylindrique (28) et les dents (36) de la denture (34) est plus longue après la ligne neutre (42) qu'avant la ligne neutre (42).

6. Dispositif de prélèvement selon l'une quelconque des revendications précédentes, caractérisé en ce que les dentures (34) des mâchoires (8) présentent chacune une butée (46) qui bloque le coulissement axial de la crémaillère cylindrique (28) dans une position de fin de course.

7. Dispositif de prélèvement selon l'une quelconque des revendications précédentes, caractérisé en ce que la pince (6) comprend au moins trois mâchoires (8).

8. Dispositif de prélèvement selon l'une quelconque des revendications précédentes, caractérisé en ce qu'il comprend une chape (20) présentant une rainure diamétrale (22) dans laquelle sont logées les branches de montage (14) des mâchoires (8).

9. Dispositif de prélèvement selon l'une quelconque des revendications précédentes, caractérisé en ce qu'une aiguille est prévue en extrémité de la crémaillère cylindrique (28).

10. Système à engrenage comprenant un pignon menant et un pignon mené, caractérisé en ce que le pignon menant est une crémaillère cylindrique (28), et en ce que le pignon mené présente sur une partie circulaire de son périmètre une denture (34) engageant la crémaillère cylindrique (28).

11. Système à engrenage selon la revendication 10, caractérisé en ce que la denture (34) engage la crémaillère cylindrique (28) par au moins deux de ses dents (36).

12. Système à engrenage selon l'une quelconque des revendications 10 ou 11, caractérisé en ce que le nombre apparent de dents (36) réparties sur le diamètre primitif (38) de la denture (34) est de six au plus.

13. Système à engrenage selon l'une quelconque des revendications 10 à 12, caractérisé en ce que la crémaillère cylindrique (28) est constituée par une succession alternée de dents annulaires (30) et de gorges annulaires (32).

14. Système à engrenage selon la revendication 13, caractérisé en ce que la ligne (40) formée par les points de contact entre les dents (30) du pignon menant (28) et les dents (36) du pignon mené (34) est plus longue après la ligne neutre (42) qu'avant là ligne neutre (42).

15. Système à engrenage selon l'une quelconque des revendications 10 à 14, caractérisé en ce que les dentures (34) présentent chacune une butée (46) qui bloque le coulissement axial de la crémaillère cylindrique (28) dans une position de fin de course.
